(19) Europäisches Patentamt
European Patent Office
Office européen des brevets

(11) **EP 4 321 156 A1**

(12) **EUROPEAN PATENT APPLICATION**
published in accordance with Art. 153(4) EPC

(43) Date of publication:
**14.02.2024 Bulletin 2024/07**

(21) Application number: **22884061.7**

(22) Date of filing: **20.10.2022**

(51) International Patent Classification (IPC):
*A61K 9/24* (2006.01)    *A61K 9/20* (2006.01)
*A61K 47/20* (2006.01)    *A61K 47/12* (2006.01)
*A61K 9/28* (2006.01)    *A61K 31/47* (2006.01)
*A61K 31/495* (2006.01)

(52) Cooperative Patent Classification (CPC):
**A61K 9/20; A61K 9/209; A61K 9/28; A61K 31/47;
A61K 31/495; A61K 47/12; A61K 47/20**

(86) International application number:
**PCT/KR2022/016031**

(87) International publication number:
**WO 2023/068839 (27.04.2023 Gazette 2023/17)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.10.2021 KR 20210141338**

(71) Applicant: **Han Wha Pharma Co., Ltd.
Chuncheon-si, Gangwon-do 24468 (KR)**

(72) Inventors:
• **CHAE, Yu Byeong**
  Suwon-si, Gyeonggi-do 16693 (KR)
• **SHIN, Seo Young**
  Suwon-si, Gyeonggi-do 16532 (KR)
• **LIM, Chae Yong**
  Suwon-si, Gyeonggi-do 16508 (KR)
• **OH, Dong Joon**
  Suwon-si Gyeonggi-do 16543 (KR)

(74) Representative: **Graf von Stosch
Patentanwaltsgesellschaft mbH
Prinzregentenstraße 22
80538 München (DE)**

(54) **FILM-COATED TABLET WITH IMPROVED STABILITY CONTAINING MONTELUKAST OR PHARMACEUTICALLY ACCEPTABLE SALT THEREOF AND LEVOCETIRIZINE OR PHARMACEUTICALLY ACCEPTABLE SALT THEREOF**

(57)    Provided are a tablet comprising: a first drug layer comprising montelukast or a pharmaceutically acceptable salt thereof; a second drug layer comprising levocetirizine or a pharmaceutically acceptable salt thereof; an inner layer being in contact with the first drug layer from one side and in contact with the second drug layer from the other side, such that the first drug layer and the second drug layer are non-contact; and an outer layer comprising the first drug layer, the inner layer, and the second drug layer, and a method for manufacturing the same.

The tablet of the present invention, in which montelukast and levocetirizine are present in a single tablet, but not contacted by a boundary layer, can be usefully used in the pharmaceutical field as a composite formulation that can be manufactured in an efficient process while minimizing drug interactions.

[FIG. 1]
Final coating (outer coating)
Coating with Levocetirizine HCl (drug coating)
Sub-coating (Inner coating)
Montelukast tablet
M

**Description**

TECHNICAL FIELD

The present invention relates to a composite formulation comprising montelukast and levocetirizine, more specifically, to a tablet comprising montelukast and levocetirizine in a non-contact state with each other.

BACKGROUND ART

Montelukast is a leukotriene antagonist and leukotriene biosynthesis inhibitor. US 5,565,473 discloses montelukast, and the use of montelukast in pulmonary diseases including asthma, bronchitis and related obstructive airway diseases; allergic responses including allergic rhinitis, contact dermatitis, allergic conjunctivitis; inflammation including arthritis or inflammatory bowel disease; skin diseases including pain, psoriasis and atopic eczema; and cardiovascular diseases including angina pectoris, myocardial ischemia, hypertension and platelet aggregation.

Cetirizine is a second-generation $H_1$ histamine receptor antagonist, and provides advantages in use over first-generation antihistamines in the racemic state. US 5,698,558 teaches that the administration of the pure optical isomer (-) cetirizine, which is levocetirizine, can reduce or prevent the side effects associated with the use of racemic cetirizine, wherein the side effects include sedation and drowsiness, headache, gastrointestinal disturbances, dizziness, nausea, arrhythmia and other effects on the cardiovascular system.

Many reports have shown that when the leukotriene inhibitor is administered in combination with cetirizine, a synergistic effect is provided for the treatment or prevention of inflammation, asthma, or allergic diseases. In addition, it has been reported that the combination can reduce the side effects related with $H_1$ histamine receptor antagonist. Accordingly, a composite formulation comprising montelukast sodium and levocetirizine hydrochloride is being developed, but since drug interactions between montelukast sodium and levocetirizine hydrochloride have been reported, various formulation studies are conducted to solve the problem.

As one of the examples, a capsule formulation prepared by preparing montelukast sodium and levocetirizine hydrochloride into individual tablets and filling them into a capsule has been reported, it is currently used in clinical practice. However, the capsule formulation has a manufacturing complexity problem because tablets should be prepared and the filled into a capsule in the manufacturing process. Therefore, there is a need for the development of a composite formulation that can be manufactured in a more efficient process while minimizing the drug interactions between montelukast sodium and levocetirizine hydrochloride.

DETAILED DESCRIPTION OF THE INVENTION

TECHNICAL PROBLEM

The problem to be solved by the present invention is to provide a composite formulation manufactured in a more efficient process while minimizing the drug interactions between montelukast and levocetirizine.

In addition, the problem solved by the present invention is to provide a method for manufacturing the composite formulation.

The problem to be solved by the present invention is not limited to the above-mentioned problems, and other technical problems that are not mentioned can be clearly understood by one of ordinary skill in the art to which the present invention belongs, from the description below.

TECHNICAL SOLUTION

In order to solve the above problems, according to an aspect of the present invention, provided is a tablet comprising a first drug layer comprising montelukast or a pharmaceutically acceptable salt thereof; a second drug layer comprising levocetirizine or a pharmaceutically acceptable salt thereof; an inner layer being in contact with the first drug layer from one side and in contact with the second drug layer from the other side, such that the first drug layer and the second drug layer are non-contact; and an outer layer comprising the first drug layer, the inner layer, and the second drug layer.

One embodiment of the present invention provides a three-layer coated tablet comprising: a core tablet comprising montelukast sodium; a drug coating layer comprising levocetirizine hydrochloride; an inner coating layer being in contact with the core tablet from one side and in contact with the drug coating layer from the other side, such that the core tablet and the drug coating layer are non-contact; and an outer coating layer comprising the core tablet, the inner coating layer, and the drug coating layer.

According to another aspect of the present invention, provided is a method for manufacturing a tablet, the method comprising: (a) preparing a first drug layer comprising montelukast or a pharmaceutically acceptable salt thereof; (b) preparing an inner layer surrounding the first drug layer; (c) preparing a second drug layer comprising levocetirizine or a pharmaceutically acceptable salt thereof and surrounding the inner layer; and (d) preparing an outer layer surrounding the second drug layer.

ADVANTAGEOUS EFFECTS

According to the present invention, it was confirmed that when the two drugs, montelukast and levocetirizine, are present in a single tablet and but are not contacted by a boundary layer by an inner coating layer (e.g., a coating layer), the two drugs maintained stability under both stress storage conditions and accelerated storage conditions without causing a significant change in the generation of related substances. In addition, it was confirmed that regardless of whether an organic acid, an acidifying agent, was included or not in the drug coating layer, there was not a significance change in the generation of related substances, and the change of the coating mechanism did not cause a significance change in the generation of related substances. In addition, the results of the dissolution test performed with the manufactured two-drug composite tablet showed that both of the active pharmaceutical ingredients (montelukast, levocetirizine) included in the coated tablet were included in the suitable ranges.

Therefore, the tablet of the present invention, in which montelukast and levocetirizine are present in a single tablet, but not contacted by a boundary layer, can be usefully used in the pharmaceutical field as a composite formulation that can be manufactured in an efficient process while minimizing drug interactions.

The effects of the present invention are not limited to the effects described above, and it should be understood to include all effects that can be inferred from the configuration of the invention described in the detailed description or claims of the present invention.

DESCRIPTION OF THE DRAWINGS

FIG. 1 is a schematic showing the structure of the tablet of the present invention.

FIG. 2a to 2d are graphs showing the results of the dissolution test of levocetirizine hydrochloride with the coated tablet of the present invention, each in water (FIG. 2a), the solution 1(pH 1.2 buffer) (FIG. 2b), pH 4.0 buffer (FIG. 2c), the solution 2(pH 6.8 buffer) (FIG. 2d).

FIG. 3a to 3d are graphs showing the results of the dissolution test of montelukast sodium with the coated tablet of the present invention, each in water (FIG. 3a), the solution 1 (pH 1.2 buffer) (FIG. 3b), pH 4.0 buffer (FIG. 3c), the solution 2 (pH 6.8 buffer) (FIG. 3d) [each including 0.5% sodium lauryl sulfate (SLS)].

MODE OF THE INVENTION

The present invention provides a tablet comprising a first drug layer comprising montelukast or a pharmaceutically acceptable salt thereof; a second drug layer comprising levocetirizine or a pharmaceutically acceptable salt thereof; an inner layer being in contact with the first drug layer from one side and in contact with the second drug layer from the other side, such that the first drug layer and the second drug layer are non-contact; and an outer layer comprising the first drug layer, the inner layer, and the second drug layer.

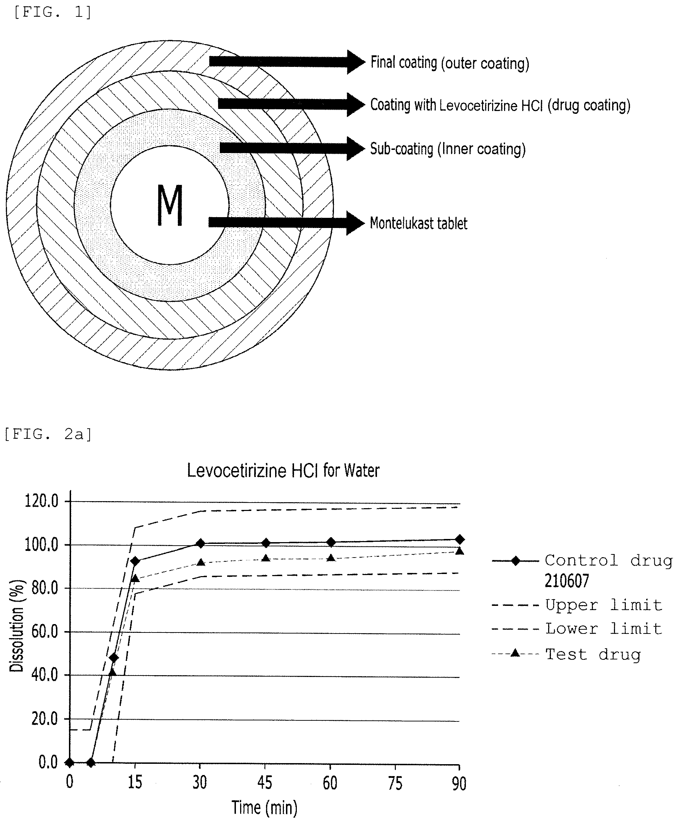

In one embodiment of the present invention, the montelukast or a pharmaceutically acceptable salt thereof may be montelukast sodium, and the levocetirizine or a pharmaceutically acceptable salt thereof may be levocetirizine hydrochloride. The tablet of the present invention preferably has a coated tablet structure comprising three coating layers, which are a core tablet, a primary inner coating (sub-coating), a secondary drug coating (coating with Levocetirizine HCl), and a tertiary outer coating (final coating), as shown in FIG. 1. In one embodiment of the present invention, as shown in FIG. 1, the first drug layer comprising montelukast (first drug) may be a core tablet comprising montelukast sodium.

In one embodiment of the present invention, the core tablet may further comprise one or more additives selected from excipient, disintegrant and lubricant. Examples of the excipient include at least one selected from lactose, microcrystalline cellulose and mannitol; examples of the disintegrant include at least one selected from croscarmellose sodium and crospovidone; and examples of the lubricant include at least one selected from colloidal silicon dioxide, talc and magnesium stearate, but are not limited thereto.

In one embodiment of the present invention, the first drug may be included in an amount of 2 wt% to 10 wt% based on the total weight of the tablet, the excipient 40 wt% to 70 wt%, the disintegrant 5 wt% to 20 wt%, and the lubricant 1 wt% % to 5 wt%.

In one embodiment of the present invention, as shown in FIG. 1, the second drug layer comprising levocetirizine (second drug) may be a drug coating layer comprising levocetirizine hydrochloride.

In one embodiment of the present invention, the drug coating layer may comprise a coating agent. As the coating agent, a coating agent comprising a polyvinyl alcohol (PVA)-polyethylene glycol (PEG) copolymer or a coating agent comprising hypromellose (HPMC) may be used, but is not limited thereto.

The coating agent comprising polyvinyl alcohol (PVA)-polyethylene glycol (PEG) copolymer may further comprise at least one ingredient selected from talc, glyceryl monocapry-locaprate, and polyvinyl alcohol. As the coating agent, commercially available Opadry QX or the like may be used, but is not limited thereto.

As the coating agent comprising hypromellose (HPMC), a commercially available Opadry hypromellose (HPMC)-based coating base may be used, but is not limited thereto.

In one embodiment of the present invention, the second drug may be included in an amount of 1 wt% to 5 wt% based on the total weight of the tablet.

In one embodiment of the present invention, the inner layer may be a coating layer. The coating layer which is the inner layer may comprise at least one coating agent selected from a coating agent comprising a polyvinyl alcohol (PVA)-polyethylene glycol (PEG) copolymer, a coating agent comprising hypromellose (HPMC), and a coating agent comprising polyvinyl alcohol (PVA), but is not limited to.

The coating agent comprising polyvinyl alcohol (PVA)-polyethylene glycol (PEG) copolymer and the coating agent comprising hypromellose (HPMC) are described above.

As the coating agent comprising polyvinyl alcohol (PVA), commercially available Opadry AMB II coating base may be used, but is not limited thereto.

In one embodiment of the present invention, the outer layer may be a coating layer. The coating layer which is the outer coating may comprise a coating agent comprising a polyvinyl alcohol (PVA)-polyethylene glycol (PEG) copolymer or a coating agent comprising hypromellose (HPMC), but is not limited thereto.

The coating agent comprising polyvinyl alcohol (PVA)-polyethylene glycol (PEG) copolymer is described above.

The coating agent comprising hypromellose (HPMC) may further comprise at least one ingredient selected from titanium oxide, talc, and polyethylene glycol 400, and may further comprise, for example, a pigment ingredient such as iron oxide yellow. As the coating agent, commercially available Opadry hypromellose (HPMC)-based coating base or hypromellose (HPMC)-based Tabshield yellow may be used, but is not limited thereto.

With regard to the coating agent used in each layer, the coating agent included in the coating layer which is the inner layer, the coating agent included in the drug coating layer which is the second drug layer, and the coating agent included in the coating layer which is the outer layer may be added in an amount of 10 wt% to 30 wt% all together based on the total weight of the tablet, and may be added by being appropriately distributed to the coating layer which is the inner layer (primary coating), the drug coating layer which is the second drug layer (secondary coating), and the coating layer which is the outer layer (tertiary coating).

In addition to excipient, disintegrant, lubricant and coating agent, the tablet may comprise additives of other ingredients according to the need within the range of 20 wt% based on the total weight of the tablet.

One preferable embodiment of the present invention provides a three-layer coated tablet comprising: a core tablet comprising montelukast sodium; a drug coating layer comprising levocetirizine hydrochloride; an inner coating layer being in contact with the core tablet from one side and in contact with the drug coating layer from the other side, such that the core tablet and the drug coating layer are non-contact; and an outer coating layer comprising the core tablet, the inner coating layer, and the drug coating layer.

The present invention also provides a method for manufacturing the tablet according to the present invention. The method for manufacturing the tablet comprises: (a) preparing a first drug layer comprising montelukast or a pharmaceutically acceptable salt thereof; (b) preparing an inner layer surrounding the first drug layer; (c) preparing a second drug layer comprising levocetirizine or a pharmaceutically acceptable salt thereof and surrounding the inner layer; and (d) preparing an outer layer surrounding the second drug layer.

Step (a) is preparing a first drug layer comprising montelukast or a pharmaceutically acceptable salt thereof, and may be carried out by a wet granulation method or direct compression method by using a combination of the first drug (preferably montelukast sodium) and an appropriate additive. The prepared first drug layer may be referred to as a core tablet or a core.

Step (b) is preparing an inner layer surrounding the first drug layer, wherein a coating solution is prepared by mixing an appropriate coating agent, purified water and so on, and the first drug layer (core tablet) comprising first drug (preferably montelukast sodium) is coated to prepare a primary coating layer (inner layer) to block the core tablet and the outside. The prepared intermediate product may be referred to as an inner coating layer tablet.

Step (c) is preparing a second drug layer comprising levocetirizine or a pharmaceutically acceptable salt thereof and surrounding the inner layer, wherein a coating solution is prepared by mixing a second drug (preferably levocetirizine hydrochloride), an appropriate coating agent, purified water and so on, and the inner coating layer tablet, which has undergone primary coating, is coated to prepare a secondary inner coating layer tablet (inner coating + drug coating) in which the first drug (preferably montelukast sodium), and the second drug (preferably levocetirizine hydrochloride) in the drug coating layer (second drug layer) are separated. Step (d) is preparing an outer layer surrounding the second drug layer, wherein a coating solution is prepared by mixing an appropriate coating agent purified water and so on, and the secondary coated tablet is coated to prepare a tertiary coating layer (outer layer) so that the second drug (preferably levocetirizine hydrochloride) in the drug coating layer may be blocked from the outside.

Hereinafter, the present invention will be described in more detail through Examples and Test Examples. However, the following Examples and Test Examples are intended to illustrate the present invention, and the scope of the present invention is not limited thereto.

**<Examples>**

**Examples 1 and 2: Manufacturing of composite tablet in which the active pharmaceutical ingredients are separated (I)**

[Table 1]

| (Unit: mg) | Example 1 | Example 2 |
|---|---|---|
| - Montelukast-comprising layer- | | |
| Montelukast sodium | 10.4 | 10.4 |
| D-mannitol | 150.0 | 150.0 |
| Microcrystalline cellulose | 15.6 | 15.6 |
| Sodium lauryl sulfate | 4.0 | 4.0 |
| Hydroxypropyl cellulose | 4.0 | 4.0 |
| 70% ethanol | (35.0) | (35.0) |
| Croscarmellose sodium | 15.0 | 15.0 |
| Colloidal silicon dioxide | 1.5 | 1.5 |
| Magnesium stearate | 2.0 | 2.0 |
| -Inner coating layer- | | |
| Opadry QX clear | 6.0 | - |
| Purified water | (24.0) | - |
| Opadry clear | - | 6.0 |
| Purified water | - | (110.0) |
| -Drug coating layer- | | |
| Levocetirizine hydrochloride | 5.0 | 5.0 |
| Opadry QX clear | 15.0 | - |
| Purified water | (100.0) | - |
| Opadry clear | - | 15.0 |
| Purified water | - | (380.0) |
| -Outer coating layer- | | |
| Opadry QX yellow | 6.0 | - |
| Purified water | (24.0) | - |
| Opadry yellow | - | 6.0 |
| Purified water | - | (110.0) |

For the montelukast-comprising layer shown in Table 1 above, wet granules were prepared by using the ingredients except the disintegrant (croscarmellose sodium) and the lubricant (colloidal silicon dioxide and magnesium stearate), and then the remaining ingredients were mixed. The resulting mixture was sieved with a 25-mesh sieve, and the prepared granules were manufactured into a tablet (core) comprising only montelukast by using a circular punch having a diameter of Φ7.5.

In addition to montelukast, a active pharmaceutical ingredient mentioned above, to manufacture a composite tablet comprising levocetirizine hydrochloride independently, the coating process described below was performed, and the specific details of the manufacturing are described below.

In Example 1, a polyvinyl alcohol (PVA)-polyethylene glycol (PEG) copolymer-based coating agent was used for all coating layers. A coating solution was prepared by using Opadry QX clear and purified water as described in the inner coating layer, and inner coating was performed on the montelukast tablet (core) to prepare a primary coated tablet (inner coating) in which the active pharmaceutical ingredient, montelukast, was blocked from the outside.

For a continuous process, while inner coating was being conducted, a coating solution was prepared by using levocetirizine hydrochloride, Opadry QX clear, purified water described in the drug coating layer. The prepared drug coating layer was used to perform drug coating on the inner coated tablet to prepare a secondary coated tablet (inner coating + drug coating) in which the active pharmaceutical ingredient in the core, montelukast, was separated from levocetirizine hydrochloride in the drug coating layer.

Outer coating was performed in order to block the levocetirizine hydrochloride of the drug coating layer, which is the outermost side of the secondary coated tablet, from the outside. Similarly, for a continuous process, while drug coating was being conducted, a coating solution was prepared by using Opadry QX yellow and purified water described in the outer coating layer of Example 1, and outer coating was performed. As a result, a three-layer coated tablet (inner coating + drug coating + outer coating) in which two active pharmaceutical ingredients were present independently in a single tablet was manufactured. In Example 2, an Opadry hypromellose (HPMC)-based coating agent was used for all coating layers, and the manufacturing method was the same as Example 1.

**Examples 3-5: Manufacturing of composite tablet in which the active pharmaceutical ingredients are separated (II)**

[Table 2]

| (Unit: mg) | Example 3 | Example 4 | Example 5 |
|---|---|---|---|
| - Montelukast-comprising layer- | | | |
| Montelukast sodium | 10.4 | 10.4 | 10.4 |
| D-mannitol | 100.0 | 100.0 | 100.0 |
| Low-substituted hydroxypropyl cellulose | 50.0 | 50.0 | 50.0 |
| Microcrystalline cellulose | 15.6 | 15.6 | 15.6 |
| Sodium lauryl sulfate | 4.0 | 4.0 | 4.0 |
| Hydroxypropyl cellulose | 2.0 | 2.0 | 2.0 |
| 70% ethanol | (25.0) | (25.0) | (25.0) |
| Croscarmellose sodium | 21.5 | 21.5 | 21.5 |
| Colloidal silicon dioxide | 3.5 | 3.5 | 3.5 |
| Magnesium stearate | 2.0 | 2.0 | 2.0 |
| -inner coating layer- | | | |
| Opadry AMB II white | 7.0 | 7.0 | 7.0 |
| Purified water | (38.0) | (38.0) | (38.0) |
| -Drug coating layer- | | | |
| Levocetirizine hydrochloride | 5.0 | 5.0 | 5.0 |
| Citric acid | - | 1.0 | 3.0 |
| Opadry clear | 10.0 | 10.0 | 10.0 |

(continued)

| -Drug coating layer- | | | |
|---|---|---|---|
| Purified water | (285.0) | (285.0) | (285.0) |
| -Outer coating layer- | | | |
| Tabshield yellow | 15.0 | 25.0 | 15.0 |
| 70% ethanol | (200.0) | (325.0) | (200.0) |

For the montelukast-comprising layer shown in Table 2 above, wet granules were prepared by the same method as Table 1. A tablet (core) comprising only montelukast was obtained by using a circular punch having a diameter of Φ7.5.

To manufacture a composite tablet comprising levocetirizine hydrochloride independently, the coating process described below was performed, and the specific details of the manufacturing are described below.

For the inner coating layer, inner coating was performed on the montelukast tablet (core) with a coating solution prepared by using polyvinyl alcohol (PVA)-based Opadry AMB II white and purified water, and a primary coated tablet (inner coating) was prepared.

For the drug coating layer, a coating solution was prepared by using levocetirizine hydrochloride, hypromellose (HPMC)-based Opadry clear and purified water. In Examples 4 and 5, citric acid, an acidifying agent, was additionally used in an amount of 1.0 mg and 3.0 mg, respectively. Drug coating was performed by using the prepared drug coating solution to manufacture a secondary coated tablet (inner coating + drug coating) in which the active pharmaceutical ingredient in the core, montelukast, was separated from levocetirizine hydrochloride in the drug coating layer.

Outer coating was performed in order to block levocetirizine hydrochloride of the drug coating layer which is the outermost side of the secondary coated tablet, from the outside. The coating solution was prepared by using the hypromellose (HPMC)-based Tabshield yellow and 70% ethanol described in the coating layer of each Example to perform outer coating.

**Examples 6 and 7: Manufacturing of composite tablet in which the active pharmaceutical ingredients are separated (I)**

[Table 3]

| (Unit: mg) | Example 6 | Example 7 |
|---|---|---|
| - Montelukast-comprising layer- | | |
| Montelukast sodium | 10.4 | 10.4 |
| D-mannitol | 100.0 | 100.0 |
| Low-substituted hydroxypropyl cellulose | 50.0 | 50.0 |
| Microcrystalline cellulose | 15.6 | 15.6 |
| Sodium lauryl sulfate | 4.0 | 4.0 |
| Hydroxypropyl cellulose | 2.0 | 2.0 |
| 70% ethanol | (25.0) | (25.0) |
| Croscarmellose sodium | 21.5 | 21.5 |
| Colloidal silicon dioxide | 3.5 | 3.5 |
| Magnesium stearate | 2.0 | 2.0 |
| -Inner coating layer- | | |
| Opadry QX white | 8.5 | 7.0 |
| Purified water | (28.0) | (38.0) |
| -Drug coating layer- | | |
| Levocetirizine hydrochloride | 5.0 | 5.0 |

(continued)

| -Drug coating layer- | | |
| --- | --- | --- |
| Citric acid | 3.0 | - |
| Acetic acid | - | 3.0 |
| Opadry clear | 10.0 | 10.0 |
| Purified water | (285.0) | (285.0) |
| -Outer coating layer- | | |
| Tabshield yellow | 15.0 | 20.0 |
| 70% ethanol | (200.0) | (200.0) |

The compositions and amounts of the montelukast-comprising layer and the outer coating layer of Examples 6 and 7 shown in Table 3 above are almost the same as or similar to those of Examples 3 to 5 shown in Table 2, and the manufacturing method is also almost the same.

Example 6 was manufactured by changing the coating base of the inner coating layer of Example 5 to polyvinyl alcohol (PVA)-polyethylene glycol (PEG) copolymer-based Opadry QX white, and Example 7 was manufactured by changing the acidifying agent of the drug coating layer of Example 6 to acetic acid.

**Examples 8-11: Manufacturing of composite tablet in which the active pharmaceutical ingredients are separated (IV)**

[Table 4]

| (Unit: mg) | Example 8 | Example 9 | Example 10 | Example 11 |
| --- | --- | --- | --- | --- |
| - Montelukast-comprising layer- | | | | |
| Montelukast sodium | 10.4 | 10.4 | 10.4 | 10.4 |
| D-mannitol | 100.0 | 100.0 | 100.0 | 100.0 |
| Low-substituted hydroxypropyl cellulose | 50.0 | 50.0 | 50.0 | 50.0 |
| Microcrystalline cellulose | 15.6 | 15.6 | 15.6 | 15.6 |
| Sodium lauryl sulfate | 4.0 | 4.0 | 4.0 | 4.0 |
| Hydroxypropyl cellulose | 2.0 | 2.0 | 2.0 | 2.0 |
| 70% ethanol | (25.0) | (25.0) | (25.0) | (25.0) |
| Croscarmellose sodium | 12.0 | 12.0 | 12.0 | 12.0 |
| Colloidal silicon dioxide | 3.5 | 3.5 | 3.5 | 3.5 |
| Magnesium stearate | 2.0 | 2.0 | 2.0 | 2.0 |
| -Inner coating layer- | | | | |
| Opadry QX clear | 6.0 | 6.0 | 6.0 | 8.0 |
| Purified water | (24.0) | (24.0) | (24.0) | (24.0) |
| -Drug coating layer- | | | | |
| Levocetirizine hydrochloride | 5.0 | 5.0 | 5.0 | 5.0 |
| Acetic acid | - | 3.0 | - | - |
| Opadry QX clear | 15.0 | 15.0 | 15.0 | 15.0 |
| Purified water | (100.0) | (115.0) | (100.0) | (100.0) |

(continued)

| -Outer coating layer- | | | | |
|---|---|---|---|---|
| Opadry QX white | 10.0 | 15.0 | 6.0 | - |
| Purified water | (24.0) | (24.0) | (24.0) | - |
| Tabshield yellow | - | - | - | 25.0 |
| 70% ethanol | - | - | - | (325.0) |
| -Final coating layer- | | | | |
| Tabshield yellow | - | - | 25.0 | - |
| 70% ethanol | - | - | (325.0) | - |

The compositions and amounts of the montelukast-comprising layer and the outer coating layer of Examples 8 to 11 shown in Table 4 above are almost the same as or similar to those of Examples 3 to 7 shown in Tables 2 and 3, and the manufacturing method is also almost the same.

In Example 8, levocetirizine hydrochloride, which was the active pharmaceutical ingredient, Opadry QX clear and purified water were used to prepare the drug coating layer, and Opadry QX white and purified water were used to prepare the outer coating layer. Polyvinyl alcohol (PVA)-polyethylene glycol (PEG) copolymer-based Opadry QX was used as the coating base of all coating layers.

In Example 9, acetic acid was added to the drug coating layer of Example 8, and the amount of Opadry QX white of the outer coating layer was increased.

In Example 10, the manufacturing method was the same as Example 8 up to the drug coating layer. The amount of Opadry QX white of the outer coating layer was reduced, and a final coating layer comprising hypromellose (HPMC)-based Tabshield yellow and 70% ethanol was included.

In Example 11, the manufacturing method was the same as Example 8 up to the drug coating layer. The outer coating layer was prepared by changing the ingredients to Tabshield yellow and 70% ethanol.

**Examples 12 and 13: Manufacturing of composite tablet in which the active pharmaceutical ingredients are separated (V)**

[Table 5]

| (Unit: mg) | Example 12 | Example 13 |
|---|---|---|
| - Montelukast-comprising layer- | | |
| Montelukast sodium | 10.4 | 10.4 |
| Croscarmellose sodium | 9.2 | 11.2 |
| Crospovidone | 13.4 | 11.4 |
| D-mannitol | 50.3 | - |
| Lactose hydrate | - | 50.3 |
| Microcrystalline cellulose | 62.0 | 62.0 |
| Colloidal silicon dioxide | 1.5 | 1.5 |
| Talc | 2.4 | 2.4 |
| Magnesium stearate | 0.8 | 0.8 |
| -Inner coating layer- | | |
| Opadry QX clear | 10.0 | 10 |
| Purified water | (40.0) | (40.0) |

(continued)

| -Drug coating layer- | | |
|---|---|---|
| Levocetirizine hydrochloride | 5.0 | 5.0 |
| Opadry QX clear | 16.0 | 15.0 |
| Purified water | (150.0) | (150.0) |
| -Outer coating layer- | | |
| Tabshield yellow | 20.0 | 20.0 |
| 70% ethanol | (200.0) | (200.0) |

In Examples 12 and 13 shown in Table 5, the composition and manufacturing method of the montelukast-comprising layer are different from those of Examples shown in Tables 1 to 4. The components except the lubricant (talc and magnesium stearate) were mixed first, and the remaining components are mixed afterwards. The resulting mixture was sieved with a No.25 Mesh sieve. The prepare granules were manufactured into a tablet (core) comprising only montelukast by using a circular punch having a diameter of Φ7.5 by the direct compression method.

Similarly, to manufacture a composite tablet comprising levocetirizine hydrochloride independently, the coating process similar to that of Example 11 was performed. Polyvinyl alcohol (PVA)-polyethylene glycol (PEG) copolymer-based Opadry QX clear and purified water were used as the coating base of both the inner coating layer and the drug coating layer. Hypromellose (HPMC)-based Tabshield yellow and 70% ethanol were used as the coating base of the outer coating layer.

**Comparative Example 1: Manufacturing of composite tablet in which the active pharmaceutical ingredients are not separated (I)**

[Table 6]

| (Unit: mg) | Comparative Example 1 |
|---|---|
| - Montelukast, Levocetirizine hydrochloride-comprising layer- | |
| Montelukast sodium | 10.4 |
| Levocetirizine hydrochloride | 5 |
| Croscarmellose sodium | 9.2 |
| Crospovidone | 13.4 |
| D-mannitol | 50.3 |
| Microcrystalline cellulose | 62.0 |
| Colloidal silicon dioxide | 1.5 |
| Talc | 2.4 |
| Magnesium stearate | 0.8 |
| -Outer coating layer- | |
| Tabshield yellow | 15.0 |
| 70% ethanol | (150.0) |

In Comparative Example 1 shown in Table 6 above, montelukast, one of the active pharmaceutical ingredients, and levocetirizine hydrochloride were mixed with other ingredients except the lubricant (talc and magnesium stearate), and then the remaining ingredients were mixed. The resulting mixture was sieved with a No.25 Mesh sieve. The prepared granules were manufactured into a composite tablet (core) comprising montelukast and levocetirizine hydrochloride by using a circular punch having a diameter of Φ7.5. After that, a coating solution was prepared by using hypromellose (HPMC)-based Tabshield yellow as a coating base together with 70% ethanol to perform primary coating (outer coating).

**Comparative Example 2: Manufacturing of composite tablet in which the active pharmaceutical ingredients are not separated (II)**

[Table 7]

| (Unit: mg) | Comparative Example 1 |
|---|---|
| - Montelukast-comprising layer- | |
| Montelukast sodium | 10.4 |
| Croscarmellose sodium | 9.2 |
| Crospovidone | 13.4 |
| D-mannitol | 50.3 |
| Microcrystalline cellulose | 62.0 |
| Colloidal silicon dioxide | 1.5 |
| Talc | 2.4 |
| Magnesium stearate | 0.8 |
| -Drug coating layer- | |
| Levocetirizine hydrochloride | 5.0 |
| Opadry QX clear | 15.0 |
| Purified water | (150.0) |
| -Outer coating layer- | |
| Tabshield yellow | 15.0 |
| 70% ethanol | (150.0) |

In Comparative Example 2 shown in Table 7 above, montelukast, which was one of the active pharmaceutical ingredients, was mixed with the ingredients except the lubricant (talc and magnesium stearate), and then the remaining ingredients were mixed. The resulting mixture was sieved with a No.25 Mesh sieve. The prepared granules were manufactured into a composite tablet (core) comprising montelukast only by using a circular punch having a diameter of Φ7.5 by the direct compression method. Then, without performing inner coating for physical separation, drug coating comprising levocetirizine hydrochloride was performed to manufacture a composite tablet a shape in which the two active pharmaceutical ingredients were presented in each layer but the boundaries were in contact with each other. After that, a coating solution was prepared by using hypromellose (HPMC)-based Tabshield yellow as a coating base together with 70% ethanol to perform outer coating.

**Test Example 1: Stress test conditions**

The composite tablets of montelukast sodium and levocetirizine hydrochloride obtained in Examples 1 to 13 and Comparative Examples 1 and 2 were subjected to stress testing for storage stability under the following conditions, and the stability was evaluated by comparing the degree of related substances of montelukast sodium and levocetirizine hydrochloride. The results are shown in Tables 11 to 12 below.

[Conditions of stress testing for storage stability]

- Test period: Initially and 1 month

- Storage condition: 60°C

- Subjects of analysis: Related substances derived from montelukast sodium and levocetirizine hydrochloride

[Conditions for analyzing montelukast sodium related substances]

(1) Preparation of test solution

Twenty or more tablets were taken, and 5 tablets out of them were broken and put into a 100 ml amber volumetric flask to which 75% ethanol was added. This solution was sonicated for 30 minutes and then stirred for 30 minute to dissolve sufficiently. After marking with 75% methanol, the solution was centrifuged at 3000 rpm for 10 minutes and filtered with a 0.45 um RC filter. The filtered solution was used as a test solution (0.5 mg/ml as montelukast).

(2) Preparation of standard solution

- Standard solution (1): The reference standard of montelukast dicyclohexylamine was precisely weighed in an amount of 13 mg and put into a 100 ml amber volumetric flask to which 75% methanol was added. After sonicating the solution, the flask was marked. The solution was precisely taken in a volume of 1 ml and put into a 100 ml amber volumetric flask to which 75% methanol was added. After marking the flask, the solution was subject to centrifugation at 3000 rpm for 10 minutes, and filtered with a 0.45 μm RC filter to prepare the standard solution (1) (0.001 mg/ml as montelukast, 0.2% compared to the test solution).
- Standard solution (2): The reference standard of sulfoxide impurity was precisely weighed in an amount of 20 mg and put into a 20 ml amber volumetric flask to which 750 methanol was added. After sonicating the mixture, the flask was marked (①). Separately, 10 mg of the reference standard of montelukast ketone impurity was precisely weighed and put into a 100 mL amber volumetric flask to which 75% methanol was added. After sonicating the solution, the flask was marked (②). Solutions ① and ② were precisely taken each in a volume of 1 ml, put into a 100 ml amber volumetric flask to which 75% methanol was added. After marking the flask, the solution was subject to centrifugation at 3000 rpm for 10 minutes, and filtered with a 0.45 μm RC filter to prepare the standard solution (2) (sulfoxide: 2% compared to the test solution, ketone: 0.2% compared to the test solution).
- Montelukast methylstyrene impurity standard solution: The reference standard of montelukast methylstyrene impurity was precisely weighed in an amount of 10 mg and put into a 100 ml amber volumetric flask to which 10 ml of DMSO and 75% methanol were added. After sonicating the mixture, the flask was marked. This solution was precisely taken in a volume of 1 ml and put into a 100 ml amber volumetric flask to which 75% methanol was added, and then the flask was marked. The retention time (RT) was confirmed and excluded from the calculation of the related substances.

(3) Operating conditions

The amount of related substances was obtained by testing and calculating the test solution and standard solution according to the following conditions.
- Mobile phase A: 0.2% (v/v) trifluoroacetic acid
- Mobile phase B: methanol: acetonitrile = 3: 2

[Table 8]

| Time (min) | Mobile phase A | Mobile phase B |
|---|---|---|
| 0 | 48 | 52 |
| 5 | 45 | 55 |
| 12 | 45 | 55 |
| 22 | 25 | 75 |
| 23 | 25 | 75 |
| 25 | 48 | 52 |
| 30 | 48 | 52 |

- Column: Luna phenyl-hexyl 4.6 x 100 mm, a 3 um or equivalent column

- Detector: UV spectrophotometer (measurement wavelength: 255 nm)

- Flow rate: 1.5 ml/min

- Column temperature: 50 °C

- Injection volume: 20 μl

<Calculation fomula 1> [Unspecified related substances]

$$\text{Unspecified related substances (\%)} = \frac{I_t}{I_s} \times \frac{C_s}{C_u} \times \frac{M_{r1}}{M_{r2}} \times P$$

* It = peak area of each related substance detected in the test solution
* $I_s$ = montelukast peak area detected in the standard solution
* $C_s$ = montelukast dicyclohexylamine standard solution concentration (mg/ml)
* $C_u$ = concentration of montelukast in the test solution (mg/ml)
* $M_{r1}$ = molecular weight of montelukast (586.18)
* $M_{r2}$ = molecular weight of montelukast dicyclohexylamine (767.50)
* P = purity (%) of montelukast dicyclohexylamine reference standard

<Calculation formula 2> [Specified related substances]

$$\text{Specified related substances (\%)} = \frac{S_t}{S_s} \times \frac{C_s}{C_u} \times P$$

* $S_t$ = peak area of each specified related substance detected in the sample solution
* $S_s$ = peak area of each specified related substance (sulfoxide impurity, montelukast ketone impurity, cis-isomer impurity) detected in the standard solution of each specified related substance
* $C_s$ = concentration of the standard solution of each specified related substance (mg/ml)
* $C_u$ = concentration of test solution (mg/ml)
* P = purity (%) of the reference standard of each specified related substance

[System suitability]

* System suitability solution: The montelukast dicyclohexylamine reference standard was precisely weighed in an amount of 13 mg, put into a 25 ml amber volumetric flask to which 75% methanol was added. This solution was sonicated and marked. The solution was accurately taken in a volume of 10 ml, put into 10 ml volumetric flask to which 4 μl of hydrogen peroxide was added. The resulting mixture was mixed well, and then exposed to light for at least 4 hours. Under these conditions, montelukast was partially converted to the cis-isomer.
* The resolution of cis-isomer and montelukast detected in the system suitability solution should be not less than 1.5.
* When injecting the standard solution 6 times, the relative standard deviation should be not more than 2%.

(4) Information about related substances

[Table 9]

| Name of related substance | Chemical name | Note |
|---|---|---|
| Sulfoxide impurity | 1-[[[1-3-[2-(7-Chloro-2-quinolinyl)ethenyl] phenyl]-3-[2-(1-hydroxy-1-methylethyl)phenyl] propyl]sulfi nyl]methyl]cyclopropaneac etic Acid | Detected with two peaks under the analytical conditions. Since the two peaks are not separated, the two peaks are integrated together. |
| Montelukast ketone impurity | (E)-1-3-[2-(7-Chloroquinolin-2-yl)vinyl]phenyl-3-[2-(2-hydroxypropan-2-yl)phenyl]propan-1-one | - |
| Cis-Isomer | 1-[[[(1R)-1-3-[(1Z)-2-(7-Chloro-2-quinolinyl) ethenyl]phenyl]-3-[2-(1-hydroxy-1-methylethyl) phenyl]propyl]thio] methyl]cyclopropaneacetic Acid | - |
| Methylstyrene impurity | [1-[[(1R)-1-3-[(E)-2-(7-chloroquinolin-2-yl) ethenyl]phenyl]-3-(2-prop-1-en-2-ylphenyl) propyl]sulfanylmethyl ]cyclopropyl]acetic acid | * RRT 1.56<br>* Excluded from the calculation (Reference: USP_Montelukast sodium tablet) |

[Conditions for analyzing levocetirizine hydrochloride related substances]

(1) Preparation of test solution

Two tablets of this drug were put into a 100 ml volumetric flask to which 10% methanol was added. After sonicating for 2 hours or more, the solution was stirred for 5 hours or more and then marked. The marked solution was subject to centrifugation at 3000 rpm for 20 minutes and filtered through a 0.45 $\mu$m PVDF (PALL) filter to prepare the test solution (0.1 mg/ml).

(2) Preparation of standard solution

- Levocetirizine hydrochloride standard solution

The levocetirizine hydrochloride reference standard was precisely weighed in an amount of 12 mg and put into a 200 ml volumetric flask to which 10% methanol was added. This solution was dissolved by shaking for 5 minutes, sonicated for 20 minutes and the marked. The solution was precisely taken in an amount of 1 ml, put into a 200 ml volumetric flask, and marked with 10% methanol. The solution filtered through a 0.45 um PVDF (PALL) filter to prepare a standard solution of levocetirizine hydrochloride (0.0003 mg/ml).

- Chlorobenzhydryl piperazine standard solution

The chlorobenzhydryl piperazine reference standard was precisely weighed in an amount of 12 mg, put into a 200 ml volumetric flask to which 10% methanol was added. This solution was shaken for 5 minutes, sonicated for 20 minutes, and then marked. The solution was precisely taken in a volume of 1 ml, put into a 20 ml volumetric flask, and marked with 10% methanol. The solution was filtered through a 0.45 um PVDF (PALL) filter to prepare a standard solution of chlorobenzhydryl piperazine. The RT (retention time) was confirmed to exclude from the related substance calculation formula.

- Levocetirizine amide standard solution

The levocetirizine amide reference standard was precisely weighed in an amount of 12 mg, put into a 200 ml volumetric flask to which 10% methanol was added. The reference standard was dissolved by shaking for 5 minutes, sonicated for 20 minutes, and then marked. The solution was precisely taken in an amount of 1 ml, put into a 20 ml volumetric flask, and marked with 10% methanol. The solution was filtered through a 0.45 $\mu$m PVDF (PALL) filter to prepare a standard solution of levocetirizine amide. The RT (retention time) was confirmed to exclude from the related substance calculation formula.

(3) Operating conditions

The test was performed with the test solutions and standard solutions by the test method described below. Related substances of which calculation result by the calculation formula was less than 0.1% was excluded from the calculation. Peaks of chlorobenzhydryl piperazine and levocetirizine amide were also excluded from the calculation.

- Mobile phase: acetonitrile : purified water : 1 M sulfuric acid = 930 : 65 : 5

- Run time: 20 minutes

- Column: Supelcosil LC-SI 4.6 x 150 mm, 5 μm or an equivalent column

- Detector: UV spectrophotometer (measurement waverength:230 nm)

- Flow rate: 1.0 ml/min

- Column temperature: 30 °C

- Injection volume: 30 μl

<Calculation Formula 3> [Impurity]

$$Impurity\,(\%) = \frac{R_U}{R_S} \times \frac{C_S}{C_U} \times P$$

* $R_U$ = peak area of each related substance detected in the test solution
* $R_S$ = peak area of levocetirizine hydrochloride detected in standard solution
* $C_S$ = concentration of levocetirizine hydrochloride in the standard solution (mg/ml)
* $C_U$ = concentration of levocetirizine hydrochloride in the test solution (mg/ml)
* $P$ = purity (%) of the levocetirizine hydrochloride reference standard

[System suitability]

*System suitability solution:

- Levocetirizine hydrochloride reference standard in an amount of 12 mg and chlorobenzhydryl piperazine reference standard in an amount of 12 mg were precisely weighed and put into a 200 ml volumetric flask, which was marked with 10% methanol and sonicated. This solution was precisely taken in an amount of 1 ml and put into a 20 ml volumetric flask, which was marked with 10% methanol. Then, this solution was filtered with a 0.45 um PVDF (PALL) filter to prepare a system suitability solution (each 3 μg/ml).

  *The resolution of levocetirizine and chlorobenzhydryl piperazine should be not less than 3.0.
  *The tailing factor of levocetirizine should be not more than 2.0.
  *When injecting the standard solution 6 times, the relative standard deviation should be not more than 2.0%.

(4) Information on related substances

[Table 10]

| Name of related substances | Chemical name | Note |
|---|---|---|
| Chlorobenzhydryl piperazine | (R)-1-[(4-Chlorophenyl)phenylmethyl]pipe razine | *Excluded from the calculation (Basis: USP_ Levocetirizine dihydrochloride tablet) |
| Levocetirizine amide | (R)-2-(2-(4-((4-chlorophe-nyl)(phenyl) methyl)pi perazin-1-yl)ethoxy)acetamide | * Excluded from the calculation (Basis: USP_ Levocetirizine di-hydrochloride tablet) |

The related substances of montelukast sodium are shown in Table 11 below, and the related substances of levocetirizine hydrochloride are shown in Table 12 below from the stress storage conditions.

[Table 11]

| Sample | Initially (%) | | | | | After 1 month under stress conditions (%) | | | | |
|---|---|---|---|---|---|---|---|---|---|---|
| | M.S | M.K | M.C.I | U.I | Total | M.S | M.K | M.C.I | U.I | Total |
| Example 1 | 0.19 | - | - | 0.05 | 0.28 | 0.89 | 0.07 | 0.03 | 0.10 | 1.42 |
| Example 2 | 0.17 | - | - | 0.03 | 0.22 | 0.75 | 0.07 | 0.03 | 0.12 | 1.12 |
| Example 3 | 0.11 | - | - | 0.07 | 0.26 | 0.59 | 0.08 | 0.08 | 0.02 | 0.98 |
| Example 4 | 0.16 | - | 0.04 | 0.04 | 0.29 | 0.72 | 0.08 | 0.08 | 0.11 | 1.52 |
| Example 5 | 0.11 | - | 0.05 | 0.05 | 0.24 | 0.47 | 0.07 | 0.09 | 0.06 | 1.67 |
| Example 6 | 0.11 | - | 0.06 | 0.05 | 0.26 | 0.57 | 0.05 | 0.08 | 0.13 | 1.47 |
| Example 7 | 0.11 | - | 0.07 | 0.04 | 0.23 | 0.53 | 0.07 | 0.10 | 0.06 | 1.40 |
| Example 8 | 0.15 | - | 0.02 | 0.10 | 0.40 | 0.69 | 0.05 | 0.07 | 0.06 | 1.00 |
| Example 9 | 0.23 | 0.04 | 0.02 | 0.07 | 0.45 | 0.46 | 0.05 | 0.08 | 0.07 | 0.98 |
| Example 10 | 0.18 | - | 0.05 | 0.06 | 0.35 | 0.71 | 0.05 | 0.10 | 0.17 | 1.43 |
| Example 11 | 0.20 | - | 0.03 | 0.05 | 0.36 | 0.73 | 0.05 | 0.09 | 0.09 | 1.10 |
| Example 12 | 0.55 | 0.01 | 0.01 | 0.03 | 0.67 | 0.80 | 0.03 | 0.08 | 0.09 | 1.19 |
| Example 13 | 0.51 | 0.03 | 0.02 | 0.03 | 0.63 | 0.85 | 0.10 | 0.07 | 0.19 | 1.21 |
| Comparative Example 1 | 0.11 | - | 0.06 | 0.05 | 0.26 | 1.21 | - | - | 1.31 | 3.65 |
| Comparative Example 2 | 0.11 | - | 0.07 | 0.04 | 0.23 | 1.64 | - | - | 0.26 | 2.65 |

*M.S : Montelukast sulfoxide
*M.K : Montelukast ketone
*M.C.I : Montelukast Cis-isomer
*U.I : Unspecified impurity

[Table 12]

| Sample | Initially (%) | | After 1 month under stress conditions (%) | |
|---|---|---|---|---|
| | U.I | Total | U.I | Total |
| Example 1 | 0.00 | 0.00 | 0.09 | 0.29 |
| Example 2 | 0.00 | 0.00 | 0.07 | 0.23 |
| Example 3 | 0.06 | 0.07 | 0.08 | 0.17 |
| Example 4 | 0.02 | 0.08 | 0.13 | 0.24 |
| Example 5 | 0.03 | 0.07 | 0.10 | 0.15 |

(continued)

| Sample | Initially (%) | | After 1 month under stress conditions (%) | |
|---|---|---|---|---|
| | U.I | Total | U.I | Total |
| Example 6 | 0.06 | 0.07 | 0.07 | 0.14 |
| Example 7 | 0.06 | 0.06 | 0.09 | 0.13 |
| Example 8 | 0.03 | 0.08 | 0.04 | 0.04 |
| Example 9 | 0.02 | 0.05 | 0.03 | 0.04 |
| Example 10 | 0.02 | 0.03 | 0.14 | 0.19 |
| Example 11 | 0.02 | 0.05 | 0.08 | 0.08 |
| Example 12 | 0.02 | 0.09 | 0.05 | 0.17 |
| Example 13 | 0.00 | 0.00 | 0.08 | 0.22 |
| Comparative Example 1 | 0.06 | 0.07 | 0.30 | 0.58 |
| Comparative Example 2 | 0.06 | 0.06 | 1.57 | 3.53 |
| *U.I : Unspecified impurity | | | | |

Tables 11 to 12 show the related substances generated under stress conditions when the two drugs of montelukast sodium and levocetirizine hydrochloride according to Examples 1-13 were present in a single tablet but formed a boundary layer by an inner coating.

Examples 4-7 and 9 implemented by using an organic acid and Examples 1-3, 8 and 10-13 implemented without using an organic acid show that there was not a significant difference in the generation of related substances.

In addition, it was confirmed that the change of the coating agent did not result in a significant difference in the generation of related substances.

Tables 11 to 12 show the related substances generated under stress conditions when the two drugs of montelukast sodium and levocetirizine hydrochloride according to Comparative Examples 1 and 2 were present in a single tablet without a boundary layer. In Comparative Example 1, in which the active pharmaceutical ingredients were mixed and contacted in the tablet, it was confirmed that among the related substances of montelukast sodium, montelukast sulfoxide and unspecified related substances were increased significantly, and among the related substances of levocetirizine hydrochloride, unspecified related substances were also increased significantly. In Comparative Example 2, in which the montelukast sodium tablet and the levocetirizine hydrochloride drug coating layer were in contact without a boundary, it was confirmed that among the related substances of montelukast sodium, montelukast sulfoxide was increased significantly, and the related substances of levocetirizine hydrochloride were increased more, compared with Comparative Example 1.

In Comparative Example 1, it was confirmed that the amount of unspecified related substances was also increased. In addition, the total amount of related substances was increased significantly. The related substances of levocetirizine hydrochloride were also increased significantly. In Comparative Example 2, it was confirmed that the amount of unspecified related substances was increased significantly.

**Test Example 2: Accelerated test conditions**

The composite tablets of montelukast sodium and levocetirizine hydrochloride obtained in Examples 1 to 13 and Comparative Examples 1 and 2 were subjected to stability testing at the accelerated storage condition under the conditions described below, and the stability was evaluated by comparing the degree of related substances of montelukast sodium and levocetirizine hydrochloride. The results are shown in Tables 13 to 14 below.

[Accelerated storage conditions]

- Test period: initially and 6 months

- Storage conditions: 40°C / RH 75%

- Analysis subject: related substances of montelukast sodium and levocetirizine hydrochloride

The related substances of montelukast sodium under the accelerated storage conditions are shown in Table 13 below, and the related substances of levocetirizine hydrochloride are shown in Table 14 below.

[Conditions for analyzing montelukast sodium related substances]

The conditions were the same as Test Example 1.

[Conditions for analyzing levocetirizine hydrochloride related substances]

The conditions were the same as Test Example 1.

The related substances of montelukast sodium under the accelerated storage conditions are shown in Table 13 below, and the related substances of levocetirizine hydrochloride are shown in Table 14 below.

[Table 13]

| Sample | Initially (%) | | | | | After 6 months under accelerated conditions(%) | | | | |
|---|---|---|---|---|---|---|---|---|---|---|
| | M.S | M.K | M.C.I | U.I | Total | M.S | M.K | M.C.I | U.I | Total |
| Example 1 | 0.19 | - | - | 0.05 | 0.28 | 0.22 | 0.02 | 0.03 | 0.03 | 0.38 |
| Example 2 | 0.17 | - | - | 0.03 | 0.22 | 0.13 | - | 0.03 | 0.09 | 0.36 |
| Example 3 | 0.11 | - | - | 0.07 | 0.26 | 0.35 | 0.05 | 0.01 | 0.06 | 0.54 |
| Example 4 | 0.16 | - | 0.04 | 0.04 | 0.29 | 0.35 | 0.03 | 0.07 | 0.07 | 0.71 |
| Example 5 | 0.11 | - | 0.05 | 0.05 | 0.24 | 0.28 | 0.05 | 0.02 | 0.05 | 0.51 |
| Example 6 | 0.11 | - | 0.06 | 0.05 | 0.26 | 0.29 | 0.03 | 0.04 | 0.04 | 0.57 |
| Example 7 | 0.11 | - | 0.07 | 0.04 | 0.23 | 0.31 | 0.04 | 0.08 | 0.08 | 0.67 |
| Example 8 | 0.15 | - | 0.02 | 0.10 | 0.40 | 0.46 | 0.05 | 0.06 | 0.06 | 0.89 |
| Example 9 | 0.23 | 0.04 | 0.02 | 0.07 | 0.45 | 0.26 | 0.03 | 0.06 | 0.04 | 0.73 |
| Example 10 | 0.18 | - | 0.05 | 0.06 | 0.35 | 0.40 | 0.03 | 0.01 | 0.07 | 0.77 |
| Example 11 | 0.20 | - | 0.03 | 0.05 | 0.36 | 0.43 | 0.03 | 0.01 | 0.08 | 0.80 |
| Example 12 | 0.55 | 0.01 | 0.01 | 0.03 | 0.67 | 0.64 | - | 0.03 | 0.08 | 0.86 |
| Example 13 | 0.51 | 0.03 | 0.02 | 0.03 | 0.63 | 0.64 | 0.03 | 0.01 | 0.10 | 1.07 |
| Comparative Example 1 | 0.11 | - | 0.06 | 0.05 | 0.26 | 1.67 | 0.15 | 0.06 | 1.47 | 3.61 |
| Comparative Example 2 | 0.11 | - | 0.07 | 0.04 | 0.23 | 0.93 | 0.05 | 0.03 | 0.34 | 1.74 |

* M.S : Montelukast sulfoxide
* M.K : Montelukast ketone
* M.C.I : Montelukast Cis-Isomer
* U.I : Unspecified impurity

[Table 14]

| Sample | Initially (%) | | After 6 months under accelerated conditions (%) | |
|---|---|---|---|---|
| | U.I | Total | U.I | Total |
| Example 1 | 0.00 | 0.00 | 0.07 | 0.21 |
| Example 2 | 0.00 | 0.00 | 0.05 | 0.19 |

(continued)

| Sample | Initially (%) | | After 6 months under accelerated conditions (%) | |
|---|---|---|---|---|
| | U.I | Total | U.I | Total |
| Example 3 | 0.06 | 0.07 | 0.03 | 0.09 |
| Example 4 | 0.02 | 0.08 | 0.11 | 0.24 |
| Example 5 | 0.03 | 0.07 | 0.10 | 0.27 |
| Example 6 | 0.06 | 0.07 | 0.13 | 0.28 |
| Example 7 | 0.06 | 0.06 | 0.07 | 0.07 |
| Example 8 | 0.03 | 0.08 | 0.08 | 0.09 |
| Example 9 | 0.02 | 0.05 | 0.04 | 0.06 |
| Example 10 | 0.02 | 0.03 | 0.07 | 0.08 |
| Example 11 | 0.02 | 0.05 | 0.18 | 0.21 |
| Example 12 | 0.02 | 0.09 | 0.09 | 0.20 |
| Example 13 | 0.00 | 0.00 | 0.02 | 0.03 |
| Comparative Example 1 | 0.06 | 0.07 | 0.24 | 0.57 |
| Comparative Example 2 | 0.06 | 0.06 | 0.83 | 1.73 |
| * U.I : Unspecified impurity | | | | |

Tables 13 to 14 show the related substances generated under the accelerated conditions when the two drugs of montelukast sodium and levocetirizine hydrochloride according to Examples 1-13 were present in a single tablet but formed a boundary layer by an inner coating.

Examples 4-7 and 9 implemented by using an organic acid and Examples 1-3, 8 and 10-13 implemented without using an organic acid show that there was not a significant difference in the generation of related substances.

In addition, it was confirmed that the change of the coating agent did not result in a significant difference in the generation of related substances.

Tables 13 to 14 show the related substances generated under the accelerated conditions when the two drugs of montelukast sodium and levocetirizine hydrochloride according to Comparative Examples 1 and 2 were present in a single tablet without a boundary layer. In Comparative Example 1, in which the active pharmaceutical ingredients were mixed and contacted in the tablet, it was confirmed that among the related substances of montelukast sodium, montelukast sulfoxide, montelukast ketone and unspecified related substances were increased, and among the related substances of levocetirizine hydrochloride, unspecified related substances were also increased significantly. In Comparative Example 2, in which the montelukast sodium tablet and the levocetirizine hydrochloride drug coating layer were in contact without a boundary, it was confirmed that among the related substances of montelukast sodium, montelukast sulfoxide and, unspecified related substances were increased significantly, and the related substances of levocetirizine hydrochloride were increased more, compared with Comparative Example 1.

**Test Example 3: Dissolution test**

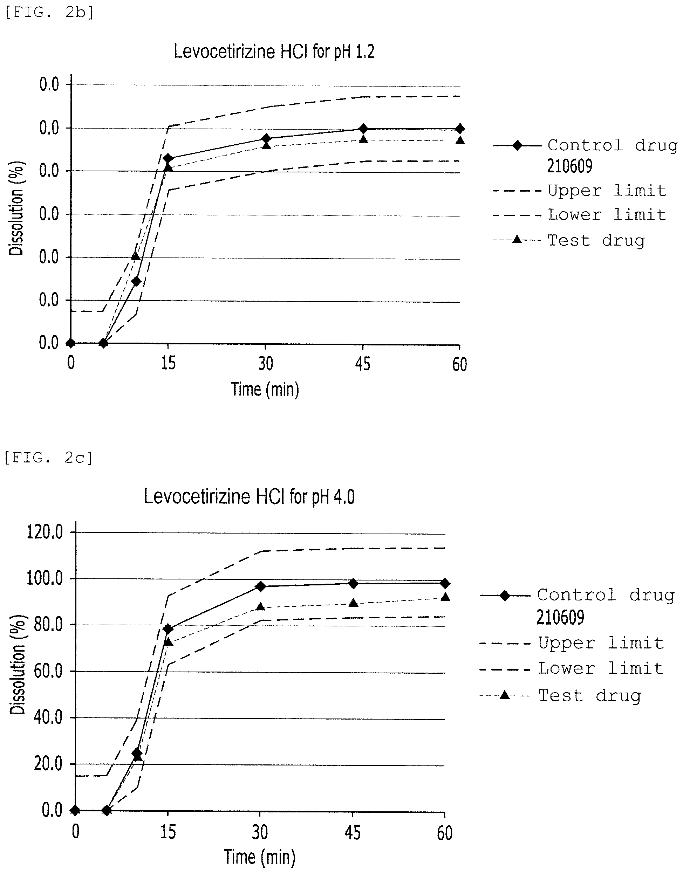

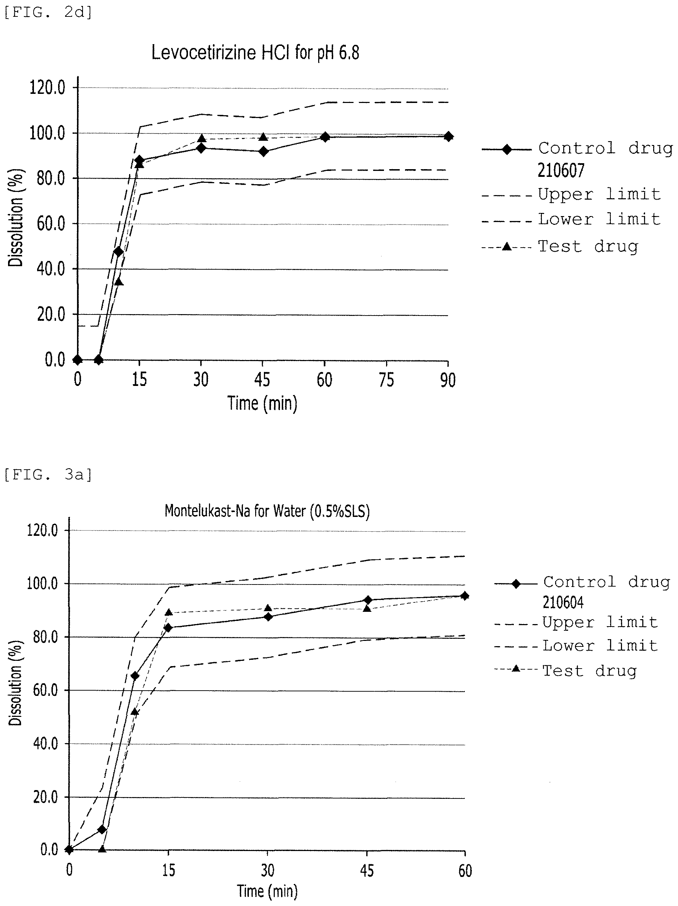

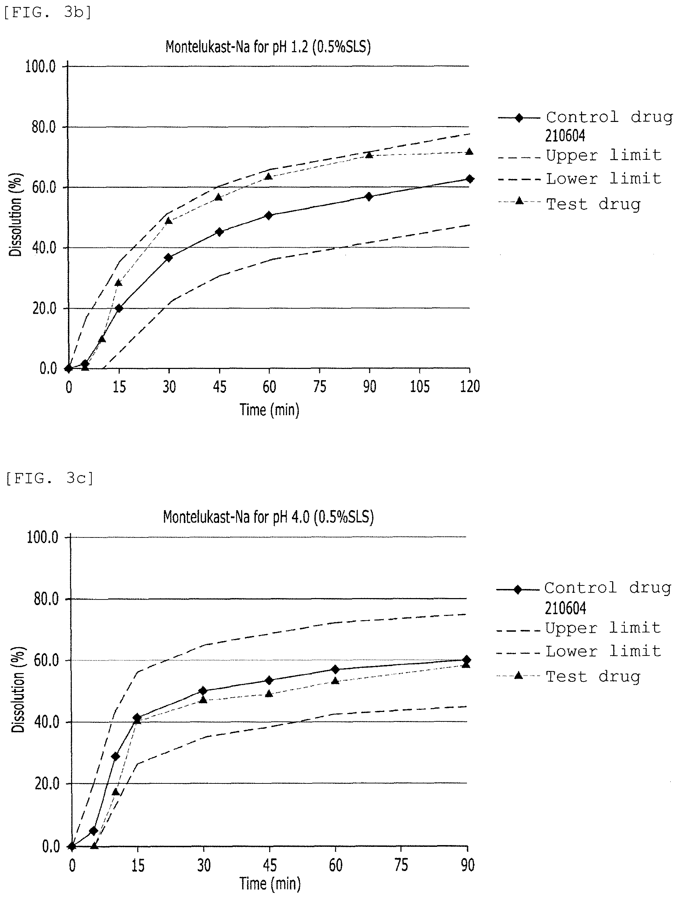

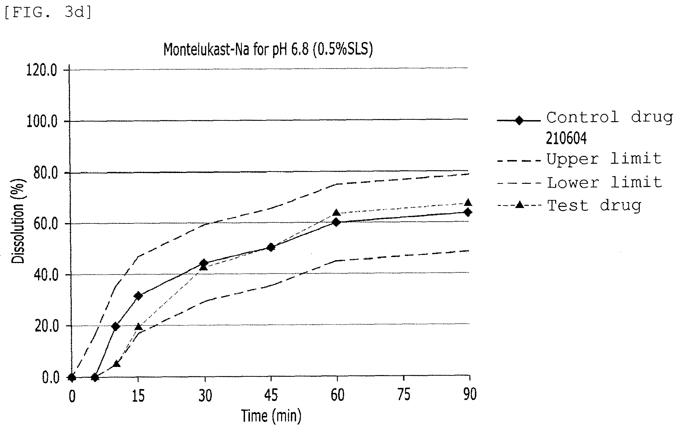

A dissolution test was performed with the coated tablet of Example 13 under the conditions described below, and the results are shown in FIGS. 2a to 2d (dissolution rate of levocetirizine) and FIGS. 3a to 3d (dissolution rate of montelukast).

<Test conditions>

- Dissolution medium: Solution 1 (pH 1.2 buffer) and Solution 2 (pH 6.8 buffer) according to the dissolution test of the Korean Pharmacopoeia, water, a pH 4.0 buffer (all the test buffer + sodium lauryl sulfate (SLS) 0.5 % added as a solubilizer in the case of montelukast sodium), 37±0.5°C

- Test method: Method 2 according to the dissolution test of the Korean Pharmacopoeia
- Test sample: control drug (monterizine capsule) and test drug (coated tablet of Example 13)

As shown in FIGS. 2a to 2d (dissolution rate of levocetirizine) and 3a to 3d (dissolution rate of montelukast), the dissolution rate of the coated tablet of Example 13 was measured based on the active pharmaceutical ingredients (montelukast and levocetirizine) of the control drug. The results confirmed that both of the active pharmaceutical ingredients (montelukast and levocetirizine) included in the coated tablet of Example 13 were included in the suitable range.

The above description of the present invention is for illustration, and those of ordinary skill in the art to which the present invention pertains can understand that it can be easily modified into other specific forms without changing the technical principle or essential features of the present invention. Therefore, it should be understood that the Examples described above are illustrative in all respects and not restrictive. For example, each component described as a single type may be implemented in a dispersed form, and likewise components described as distributed may be implemented in a combined form.

The scope of the present invention is represented by the following claims, and all changes or modifications derived from the meaning and scope of the claims and their equivalents should be construed as being included in the scope of the present invention.

**Claims**

1. A tablet comprising:

   a first drug layer comprising montelukast or a pharmaceutically acceptable salt thereof;
   a second drug layer comprising levocetirizine or a pharmaceutically acceptable salt thereof;
   an inner layer being in contact with the first drug layer from one side and in contact with the second drug layer from the other side, such that the first drug layer and the second drug layer are non-contact; and
   an outer layer comprising the first drug layer, the inner layer, and the second drug layer.

2. The tablet according to claim 1, wherein the montelukast or a pharmaceutically acceptable salt thereof is montelukast sodium, and the levocetirizine or a pharmaceutically acceptable salt thereof is levocetirizine hydrochloride.

3. The tablet according to claim 1, wherein the first drug layer is a core tablet comprising montelukast sodium.

4. The tablet according to claim 3, wherein the core tablet further comprises one or more additives selected from excipient, disintegrant and lubricant.

5. The tablet according to claim 1, wherein the second drug layer is a drug coating layer comprising levocetirizine hydrochloride.

6. The tablet according to claim 1, wherein the inner layer is a coating layer.

7. The tablet according to claim 6, wherein the coating layer comprises one or more coating agent selected from a coating agent comprising a polyvinyl alcohol (PVA)-polyethylene glycol (PEG) copolymer, a coating agent comprising hypromellose (HPMC), and a coating agent comprising polyvinyl alcohol (PVA).

8. The tablet according to claim 1, wherein the outer layer is a coating layer.

9. A three-layer coated tablet comprising: a core tablet comprising montelukast sodium;

   a drug coating layer comprising levocetirizine hydrochloride;
   an inner coating layer being in contact with the core tablet from one side and in contact with the drug coating layer from the other side, such that the core tablet and the drug coating layer are non-contact; and
   an outer coating layer comprising the core tablet, the inner coating layer, and the drug coating layer.

10. A method for manufacturing a tablet, comprising:

    (a) preparing a first drug layer comprising montelukast or a pharmaceutically acceptable salt thereof;
    (b) preparing an inner layer surrounding the first drug layer;

(c) preparing a second drug layer comprising levocetirizine or a pharmaceutically acceptable salt thereof and surrounding the inner layer; and
(d) preparing an outer layer surrounding the second drug layer.

[FIG. 1]

Final coating (outer coating)

Coating with Levocetirizine HCl (drug coating)

Sub-coating (Inner coating)

Montelukast tablet

[FIG. 2a]

Levocetirizine HCl for Water

[FIG. 2b]

**Levocetirizine HCl for pH 1.2**

[FIG. 2c]

**Levocetirizine HCl for pH 4.0**

[FIG. 2d]

Levocetirizine HCl for pH 6.8

[FIG. 3a]

Montelukast-Na for Water (0.5%SLS)

[FIG. 3b]

Montelukast-Na for pH 1.2 (0.5%SLS)

[FIG. 3c]

Montelukast-Na for pH 4.0 (0.5%SLS)

[FIG. 3d]

Montelukast-Na for pH 6.8 (0.5%SLS)

Legend:
- Control drug 210604
- Upper limit
- Lower limit
- Test drug

X-axis: Time (min)
Y-axis: Dissolution (%)

| **INTERNATIONAL SEARCH REPORT** | International application No. |
|---|---|
| | **PCT/KR2022/016031** |

**A.    CLASSIFICATION OF SUBJECT MATTER**

**A61K 9/24**(2006.01)i; **A61K 9/20**(2006.01)i; **A61K 47/20**(2006.01)i; **A61K 47/12**(2006.01)i; **A61K 9/28**(2006.01)i; **A61K 31/47**(2006.01)i; **A61K 31/495**(2006.01)i

According to International Patent Classification (IPC) or to both national classification and IPC

**B.    FIELDS SEARCHED**

Minimum documentation searched (classification system followed by classification symbols)

A61K 9/24(2006.01); A61K 31/00(2006.01); A61K 31/47(2006.01); A61K 31/495(2006.01); A61K 31/496(2006.01); A61K 9/20(2006.01); A61K 9/48(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 몬테루카스트(montelukast), 레보세티리진(levocetirizine), 약물층(drug layer), 내층(inner layer), 외층(outer layer), 코어(core), 코팅(coating), 정제(tablet)

**C.    DOCUMENTS CONSIDERED TO BE RELEVANT**

| Category* | Citation of document, with indication, where appropriate, of the relevant passages | Relevant to claim No. |
|---|---|---|
| Y | WO 2012-064305 A2 (BILGIC, Mahmut) 18 May 2012 (2012-05-18)<br>    See claims 1, 11 and 12. | 1-10 |
| Y | US 2010-0172985 A1 (KAMATH, Satish et al.) 08 July 2010 (2010-07-08)<br>    See paragraphs [0016] and [0042]; example 9; claims 1, 2, 9, 11 and 17; and table 5. | 1-10 |
| A | KR 10-2019-0003323 A (HANMI PHARM. CO., LTD.) 09 January 2019 (2019-01-09)<br>    See entire document. | 1-10 |
| A | KR 10-2013-0081013 A (HANMI PHARM. CO., LTD.) 16 July 2013 (2013-07-16)<br>    See entire document. | 1-10 |
| A | US 2019-0125679 A1 (UNIVERSITY OF STRATHCLYDE) 02 May 2019 (2019-05-02)<br>    See entire document. | 1-10 |

☐ Further documents are listed in the continuation of Box C.         ☑ See patent family annex.

| *    Special categories of cited documents: | "T"    later document published after the international filing date or priority date and not in conflict with the application but cited to understand the principle or theory underlying the invention |
|---|---|
| "A"    document defining the general state of the art which is not considered to be of particular relevance | |
| "D"    document cited by the applicant in the international application | "X"    document of particular relevance; the claimed invention cannot be considered novel or cannot be considered to involve an inventive step when the document is taken alone |
| "E"    earlier application or patent but published on or after the international filing date | |
| "L"    document which may throw doubts on priority claim(s) or which is cited to establish the publication date of another citation or other special reason (as specified) | "Y"    document of particular relevance; the claimed invention cannot be considered to involve an inventive step when the document is combined with one or more other such documents, such combination being obvious to a person skilled in the art |
| "O"    document referring to an oral disclosure, use, exhibition or other means | |
| "P"    document published prior to the international filing date but later than the priority date claimed | "&"    document member of the same patent family |

| Date of the actual completion of the international search | Date of mailing of the international search report |
|---|---|
| **31 January 2023** | **01 February 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

<table>
<tr><td colspan="2">INTERNATIONAL SEARCH REPORT<br>Information on patent family members</td><td colspan="2">International application No.<br><br>**PCT/KR2022/016031**</td></tr>
</table>

| Patent document cited in search report | | | Publication date (day/month/year) | Patent family member(s) | | | Publication date (day/month/year) |
|---|---|---|---|---|---|---|---|
| WO | 2012-064305 | A2 | 18 May 2012 | WO | 2012-064301 | A2 | 18 May 2012 |
| | | | | WO | 2012-064301 | A3 | 19 July 2012 |
| | | | | WO | 2012-064305 | A3 | 09 August 2012 |
| US | 2010-0172985 | A1 | 08 July 2010 | US | 8377475 | B2 | 19 February 2013 |
| | | | | WO | 2010-078543 | A1 | 08 July 2010 |
| KR | 10-2019-0003323 | A | 09 January 2019 | KR | 10-2110304 | B1 | 14 May 2020 |
| | | | | WO | 2019-004776 | A2 | 03 January 2019 |
| | | | | WO | 2019-004776 | A3 | 11 April 2019 |
| KR | 10-2013-0081013 | A | 16 July 2013 | CN | 104039314 | A | 10 September 2014 |
| | | | | CN | 104039314 | B | 14 July 2017 |
| | | | | EP | 2800558 | A1 | 12 November 2014 |
| | | | | EP | 2800558 | A4 | 12 August 2015 |
| | | | | EP | 2800558 | B1 | 11 April 2018 |
| | | | | JP | 2015-503579 | A | 02 February 2015 |
| | | | | JP | 6163165 | B2 | 12 July 2017 |
| | | | | KR | 10-1418404 | B1 | 10 July 2014 |
| | | | | KR | 10-1843086 | B1 | 28 March 2018 |
| | | | | KR | 10-2014-0066142 | A | 30 May 2014 |
| | | | | US | 2014-0356422 | A1 | 04 December 2014 |
| | | | | US | 9486528 | B2 | 08 November 2016 |
| | | | | WO | 2013-103262 | A1 | 11 July 2013 |
| US | 2019-0125679 | A1 | 02 May 2019 | EP | 2542227 | A2 | 09 January 2013 |
| | | | | EP | 2542227 | B1 | 22 November 2017 |
| | | | | JP | 2013-521334 | A | 10 June 2013 |
| | | | | JP | 2016-026227 | A | 12 February 2016 |
| | | | | JP | 6162197 | B2 | 12 July 2017 |
| | | | | US | 10137091 | B2 | 27 November 2018 |
| | | | | US | 11065205 | B2 | 20 July 2021 |
| | | | | US | 2013-0017262 | A1 | 17 January 2013 |
| | | | | WO | 2011-107755 | A2 | 09 September 2011 |
| | | | | WO | 2011-107755 | A3 | 03 May 2012 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5565473 A
- US 5698558 A